# EUROPEAN PATENT SPECIFICATION

(11) **EP 4 346 971 B1**
(45) Date of publication and mention of the grant of the patent: **25.11.2026**
(21) Application number: 22815478.7
(22) Date of filing: 03.06.2022
(51) Int. Cl.: A61M 16/16, A61M 16/00, A61M 16/10

(54) **IMPROVED RESPIRATORY SUPPORT APPARATUS**
VERBESSERTE ATEMUNTERSTÜTZUNGSVORRICHTUNG
APPAREIL D'ASSISTANCE RESPIRATOIRE AMÉLIORÉ

(30) Priority: 04.06.2021 US 202163196856 P
(43) Date of publication of application: 10.04.2024
(73) Proprietor: Fisher & Paykel Healthcare Limited, Auckland, 2013 (NZ)
(72) Inventor: SANSON, Samuel Carey Mathew, East Tamaki, 2013 (NZ); BURGESS, Russel William, East Tamaki, 2013 (NZ)
(74) Representative: Forresters IP LLP
(86) International application number: PCT/IB2022/055175
(87) International publication number: WO 2022/254383

(56) References cited:
- WO-A1-2020/185100
- AU-A1- 2012 216 775
- CN-A- 111 150 917
- US-A1- 2011 120 462
- US-A1- 2014 116 433
- US-A1- 2014 202 460
- US-B2- 10 449 323

## Description

### FIELD OF THE DISCLOSURE

The present disclosure relates to methods and systems for providing respiratory support to a patient. In particular, the present disclosure relates to methods and systems that can reduce the risk that gases provided to the patient during a respiratory support session will have an enthalpy and/or a dew point above a threshold.

### BACKGROUND

Respiratory support devices (also known as respiratory support apparatuses or breathing assistance apparatuses) are used to deliver a flow of gases to patients. In this context, a patient may include any person receiving respiratory support from a respiratory support device (i.e. a breathing assistance apparatus). Respiratory support devices are used in various environments such as, for example, hospitals, medical facilities, residential care facilities, and the home. The flow of gases may include air, or air with supplementary oxygen (or other supplementary gases).

A respiratory support device may be a humidifier (which can heat and humidify the gases flow), may be a humidifier that can be coupled to a flow generator, or may be an integrated humidifier and flow generator. The flow generator may provide the flow of gases, and the humidifier may heat and humidify the flow of gases. A respiratory support device may allow adjustment and control over characteristics of the gases flow, including flow rate, temperature, gases concentration, humidity, and pressure, etc. Sensors, such as flow sensors and/or pressure sensors, may be used to measure characteristics of the gases flow.

A respiratory support device may heat and humidify a flow of gases via a heater plate connected to a humidifier. Control of the heater plate can help to ensure that breathing gases are delivered to the patient at the desired temperature and humidity.

US patent application publication 2014/0116433 and Australian patent application publication 2012216775 disclose systems for surgical humidification control. Some embodiments provide for humidifier control systems and methods configured to adjust power to a heater plate in a surgical humidifier to account for changes in flow rate to provide a consistent output, to provide functionality for different modes of use, and to provide accurate control over temperature and/or humidity at relatively low flows.

Chinese patent application publication 111150917 discloses a humidification control system and method for a ventilation therapy apparatus. A first controller makes the relative humidity of a current first mixed airflow, which is obtained after water vapor in a water tank is mixed with air input by the fan, at a target humidity. According to the control method, the airflow inhaled by a patient is a mixed airflow conforming to a target temperature and a target humidity.

### SUMMARY

The invention is defined in the appended independent claims 1 and 12. Preferred embodiments are matter of the dependent claims.

A respiratory support system can include a respiratory support device coupled to an inspiratory tube, which in turn can be coupled to a patient interface. In certain situations, a patient can experience discomfort and/or be harmed by the gases provided by a respiratory support system. Such discomfort and/or harm can sometimes occur even if the temperature of the gases flow is controlled below a limit. This is because comfort and safety are a function of, not just the temperature of the gases flow, but a combination of parameters, including temperature, humidity, and flow rate. Therefore, when designing safety algorithms for respiratory support systems, it is important to incorporate parameters that represent more than just gas temperature. Two such parameters are enthalpy and dew point (i.e. humidity).

The respiratory support systems disclosed herein can help to reduce the risk that the enthalpy and/or the dew point of the gases provided to the patient will exceed respective predetermined thresholds. More specifically, the systems disclosed herein can protect the patient from discomfort and/or harm by reducing the risk that the gases provided to the patient will have an undesirable, dangerous, and/or harmful enthalpy and/or dew point (that is, an enthalpy and/or dew point that exceeds the predetermined threshold).

Enthalpy can be defined as a thermodynamic quantity equivalent to the total heat content of a system. Non-limiting factors that affect the enthalpy and/or the dew point of the delivered gases include temperature and humidity. Flow rate can also be another factor that affects enthalpy. The systems disclosed herein help to maintain the enthalpy and/or the dew point of the gases (at the patient end of the system) below respective, predetermined thresholds, thereby helping to prevent discomfort or, in some cases, harm to the patient. As described herein, the respiratory support system may not directly measure the heat and humidity of the gases provided to the patient. The respiratory support system also may not directly measure the enthalpy and dew point of the gases provided to the patient.

In some situations, the heating and humidification of the gases may cause the enthalpy and/or the dew point of the gases at the patient interface to exceed a threshold that can cause discomfort and/or injury to the patient. Such situations may include, for example, when a user initiates a new respiratory support session and/or changes set point parameters during a respiratory support session.

One way to reduce the risk, that the enthalpy and/or the dew point of the gases provided to the patient will exceed respective predetermined thresholds, is to specify a single maximum heater plate temperature. The controller can then be programmed to not allow the heater plate to exceed this temperature (for example, by controlling power supplied to the heater plate such that the heater plate temperature does not exceed the maximum heater plate temperature). However, for devices with a wide range of selectable set points, one maximum heater plate temperature may not be appropriate for all selected set points. For example, a maximum heater plate temperature that appropriately limits the dew point at the patient interface during operation with high temperature and flow set points, may allow an excessively high dew point at the patient interface during operation with a high temperature set point and a low flow set point. Conversely, a maximum heater plate temperature that appropriately limits the dew point at the patient interface during operation with low temperature and flow set points, may prevent the device from achieving high temperature and flow set points, which can make the respiratory support session less effective for the patient.

Instead of specifying a single maximum heater plate temperature, the risk of patient discomfort and/or harm, due to an excessively high dew point and/or enthalpy at the patient interface, can be mitigated by setting a maximum heater plate temperature based on parameters that are determined from sensor data during operation of the respiratory support system. A dynamic maximum heater plate temperature can help to ensure that the heat applied to the breathing gases does not cause the enthalpy and/or the dew point of the breathing gases provided to the patient to exceed the threshold, while still allowing the device to achieve a wide range of set points. The maximum heater plate temperature may be automatically adjusted in real time based at least in part on the sensor data. The temperature of the heater plate is a function of the power supplied to the heater plate. The term "power" as used in the present disclosure should be understood broadly to refer to an amount of energy per unit of time. Power may be supplied in accordance with a duty cycle, current, voltage, via pulse-width modulation, via a voltage regulator, or the like.

The present disclosure relates to a respiratory support system, which includes a respiratory support device. The controller of a respiratory support device can, for any respiratory support session, use multiple parameters determined from sensor data to dynamically calculate the maximum heater plate temperature for the selected set points, and, therefore, mitigate harm without unduly restricting the range of achievable set points. The sensor data may be updated in real time and may include, but is not limited to, flow rate, pressure in the gas path, and ambient temperature. The sensor data may be used to determine dew point and humidity at the inlet to the humidification chamber. The controller can use the dynamic, maximum heater plate temperature to calculate a dynamic, maximum heater plate power. This dynamic, maximum heater plate power can help the controller to prevent patient discomfort and/or harm (caused by excessively high dew point at the patient interface) without unduly restricting the range of achievable set points.

As used herein, a user may include, for example, the patient, a nurse, a doctor, a caretaker, or any other operator of the respiratory support device. The patient may be anyone receiving therapy from the respiratory support system.

The present invention provides a respiratory support device forming part of a respiratory support system which delivers a flow of gases to a patient, wherein the respiratory support device is configured to receive a humidification chamber containing a volume of liquid. The respiratory support device comprises: a heater plate configured to transfer heat to the humidification chamber, wherein an inlet of the humidification chamber is configured to receive the flow of gases to be heated and humidified, and an outlet of the humidification chamber is configured to be coupled to an inspiratory tube; a first sensor configured to measure a current temperature of the heater plate; at least one or more other sensors configured to measure parameters relating to at least one of the gases flowing through the device, an ambient environment, or hardware within the device, wherein each of the at least one or more other sensors is configured to measure a different parameter than the rest of the at least one or more other sensors; a user interface configured to receive user input; and a controller in electrical communication with the heater plate, the first and at least one or more other sensors, and the user interface, wherein the controller is configured to control a heat output of the heater plate in order to adjust the heat transferred to the humidification chamber, wherein the controller is further configured to: determine a heater plate temperature limit in response to the user input received via the user interface and based, at least in part, on the parameters measured by the at least one or more other sensors; and calculate a maximum power limit to the heater plate based, at least in part, on the heater plate temperature limit and data from the first sensor, wherein the maximum heater plate power limit prevents the heater plate temperature from exceeding the heater plate temperature limit.

In a configuration, the heater plate temperature limit can be temperature at which the heater plate is configured to operate such that a dew point of the gases is below a limit that can result in injury to the patient.

In a configuration, the user input can comprise at least one of: a temperature set point change, a dew point set point change, a flow rate set point change, or any combination thereof.

In a configuration, the controller can be configured to update a current maximum power limit to the heater plate to be the calculated maximum power limit to the heater plate in response to the determined heater plate temperature limit being lower than a current heater plate temperature limit.

In a configuration, the controller can be configured to cut (i.e. disable) power to the heater plate, and either cut (i.e. disable) power to the inspiratory tube or reduce power to the inspiratory tube in response to the device failing to update the current maximum power limit to the heater plate to be the calculated maximum power limit to the heater plate, optionally within a threshold time. Controlling power to the inspiratory tube as described herein means controlling power provided to the inspiratory tube heater. The inspiratory tube comprises a heater (i.e. heating element) within the tube, such as for example one or more heater wires that are embedded in the walls of the tube. The embedded heater wires heat the gases passing through the tube. The heater of the inspiratory tube helps to reduce condensate forming in the inspiratory tube.

In a configuration, the controller can be configured to cut (i.e. disable) power to the heater plate and/or cut power to the inspiratory tube (i.e. inspiratory tube heater) in response to the current heater plate temperature exceeding the determined heater plate temperature limit by: 5°C for 10 minutes, or 10°C for 10 seconds, and/or in response to a current power supplied to the heater plate exceeding the determined maximum power limit to the heater plate by a statistical variation based threshold (e.g., if the current power supplied to the heater plate exceeds the determined maximum power limit to the heater plate by 3 standard deviations) .

**In** a configuration, the controller can be configured to reduce or cut (i.e. disable) power to a flow generator of the device in response to: the device failing to update the current maximum power limit to the heater plate to be the calculated maximum power limit to the heater plate, optionally within the threshold time; the current heater plate temperature exceeding the determined heater plate temperature limit; and/or the current power supplied to the heater plate exceeding the determined maximum power limit to the heater plate.

**In** a configuration, the controller can be configured to maintain an existing maximum power limit to the heater plate unless the determined heater plate temperature limit is lower than the current heater plate temperature limit.

**In** a configuration, the current heater plate temperature limit can be a pre-set temperature limit.

**In** a configuration, the pre-set temperature limit can be a temperature limit above which a hardware safety feature is triggered.

**In** a configuration, the respiratory support device further can comprise a flow generator.

**In** a configuration, the flow generator can be located in a housing of the device.

**In** a configuration, the flow generator can comprise a blower.

**In** a configuration, the power provided to the heater plate can be dependent on a duty cycle.

**In** a configuration, the outlet of the humidification chamber can comprise a removable outlet elbow.

**In** a configuration, the inspiratory tube can be heated.

A respiratory support system can comprise: the respiratory support device of any of the configurations disclosed herein; and the inspiratory tube.

**In** a configuration, the parameters measured by the at least one or more other sensors can comprise at least one of: a flow rate of the gases within a flow path of the system, a temperature measured at the outlet of the humidification chamber, a dew point measured at the outlet of the humidification chamber, a temperature measured at the inlet of the humidification chamber, a dew point measured at the inlet of the humidification chamber, an ambient temperature, a pressure of the gases within the flow path of the system, or an electrical power, current, or voltage supplied to the heater plate.

**In** a configuration, the at least one or more other sensors can comprise two or more other sensors.

**In** a configuration, the inspiratory tube can be a heated inspiratory tube.

**In** a configuration, the controller can be configured to control a heat output to a heating element in the inspiratory tube.

**In** a configuration, the respiratory support system further can comprise the humidification chamber.

**In** a configuration, the flow rate of the gases can be a mass flow rate.

**In** a configuration, the flow rate of the gases can be a volumetric flow rate.

**In** a configuration, the respiratory support system further can comprise an unsealed patient interface.

**In** a configuration, the respiratory support system further can comprise at least one of a temperature sensor or dew point sensor at or near a patient end of the inspiratory tube.

The present invention provides a method of controlling a respiratory support device, wherein the respiratory support device forms part of a respiratory support system and is configured to receive a humidification chamber to heat and humidify the flow of gases. The method comprises: under control of a controller of the respiratory support device, receiving data from a first sensor and at least two other sensors, the first sensor configured to measure a current temperature of a heater plate of the respiratory support device and the at least two other sensors configured to measure parameters relating to at least one of the gases flowing through the device, an ambient environment, or hardware within the device, wherein each of the at least two other sensors is configured to measure a different parameter than the rest of the at least two or more sensors; receiving user input, via a user interface, wherein the user input changes at least one parameter setting for the respiratory support device; determining a heater plate temperature limit in response to the user input received via the user interface and based, at least in part, on data from the at least two other sensors; and calculating a maximum power limit to the heater plate based, at least in part, on the heater plate temperature limit and the data from the first sensor, wherein the maximum heater plate power limit prevents the heater plate temperature from exceeding the heater plate temperature limit.

In a configuration, the power provided to the heater plate can be dependent on a duty cycle.

In a configuration, the heater plate temperature limit can be a temperature at which the heater plate is configured to operate without a dew-point temperature of the gases directed into the user's airway exceeding a value that can result in injury to the patient.

In a configuration, the user input can comprise at least one of: a temperature set point change, a dew point set point change, or a flow rate set point change.

In a configuration, the method further can comprise updating a current maximum power limit to the heater plate to be the calculated maximum power limit to the heater plate in response to the determined heater plate temperature limit being lower than a current heater plate temperature limit.

In a configuration, the method further can comprise disabling power to the heater plate, and either disabling or reducing power to an inspiratory tube (i.e. inspiratory tube heater) of the device in response to the device failing to update the current maximum power limit to the heater plate to be the calculated maximum power limit to the heater plate, optionally within a threshold time.

In a configuration, the method further can comprise disabling power to the heater plate and/or disabling power to the inspiratory tube (i.e. inspiratory tube heater) in response to the current heater plate temperature exceeding the determined heater plate temperature limit by: 5°C for 10 minutes, or 10°C for 10 seconds, and/or in response to a current power supplied to the heater plate exceeding the determined maximum power limit to the heater plate by a statistical variation based threshold (e.g., if the current power supplied to the heater plate exceeds the determined maximum power limit to the heater plate by 3 standard deviations).

In a configuration, the method further can comprise reducing or disabling power to a flow generator of the device in response to: the device failing to update the current maximum power limit to the heater plate to be the calculated maximum power limit to the heater plate, optionally within the threshold time; the current heater plate temperature exceeding the determined heater plate temperature limit; and/or the current power supplied to the heater plate exceeding the determined maximum power limit to the heater plate.

In a configuration, the method further can comprise maintaining an existing maximum power limit to the heater plate unless the determined heater plate temperature limit is lower than the current heater plate temperature limit.

In a configuration, the current heater plate temperature limit can be a pre-set temperature limit.

In a configuration, the pre-set temperature limit can be a temperature limit above which a hardware safety feature is triggered.

In a configuration, the parameters measured by the at least two other sensors can comprise at least two of: a flow rate of the gases within a flow path of the respiratory support system, a temperature measured at an outlet of the humidification chamber, a temperature measured at the inlet of the humidification chamber, an ambient temperature, a pressure of the gases within the flow path of the system, or an electrical power, current, or voltage supplied to the heater plate.

In a configuration, the flow rate of the gases can be a mass flow rate.

In a configuration, the flow rate of the gases can be a volumetric flow rate.

Disclosed, but not independently claimed, is a method of controlling a respiratory support device, wherein the respiratory support device is configured to receive a humidification chamber to heat and humidify the flow of gases. The method can comprise: under control of a controller of the device, calculating a flow rate of the flow of gases entering the humidification chamber based, at least in part, on operating parameters of the respiratory support device; calculating a maximum safe mass evaporation rate based, at least in part, on the calculated flow rate; and calculating a heater plate temperature limit based, at least in part, on the calculated maximum safe mass evaporation rate.

**In** a configuration, the method further can comprise determining a maximum power limit to the heater plate based, at least in part, on the heater plate temperature limit and a current heater plate temperature.

**In** a configuration, the method further can comprise: comparing the calculated heater plate temperature limit with a current heater plate temperature limit, and updating the current maximum power limit to the heater plate to be the determined maximum power limit to the heater plate in response to the calculated heater plate temperature limit being lower than the current heater plate temperature limit.

**In** a configuration, the method further can comprise disabling power to the heater plate, and either disabling or reducing power to an inspiratory tube (i.e. inspiratory tube heater) of the device in response to the device failing to update the current maximum power limit to the heater plate to be the calculated maximum power limit to the heater plate, optionally within a threshold time.

**In** a configuration, the method further can comprise disabling power to the heater plate and/or disabling power to the inspiratory tube (i.e. inspiratory tube heater) in response to the current heater plate temperature exceeding the determined heater plate temperature limit by: 5°C for 10 minutes, or 10°C for 10 seconds, and/or in response to a current power supplied to the heater plate exceeding the determined maximum power limit to the heater plate by a statistical variation based threshold (e.g., if the current power supplied to the heater plate exceeds the determined maximum power limit to the heater plate by 3 standard deviations).

**In** a configuration, the method further can comprise reducing or disabling power to a flow generator of the device in response to: the device failing to update the current maximum power limit to the heater plate to be the calculated maximum power limit to the heater plate, optionally within the threshold time; the current heater plate temperature exceeding the determined heater plate temperature limit; and/or the current power supplied to the heater plate exceeding the determined maximum power limit to the heater plate.

**In** a configuration, the method further can comprise maintaining an existing maximum power limit to the heater plate unless the calculated heater plate temperature limit is lower than the current heater plate temperature limit.

**In** a configuration, the method further can comprise increasing the maximum power limit in response to the calculated heater plate temperature limit being higher than the current heater plate temperature limit.

**In** a configuration, the current heater plate temperature limit can be a pre-set temperature limit.

**In** a configuration, the pre-set temperature limit can be a temperature limit above which a hardware safety feature is triggered.

**In** a configuration, the method further can comprise calculating a maximum safe temperature of a liquid in the humidification chamber based, at least in part, on the calculated flow rate.

**In** a configuration, the method further can comprise calculating a maximum safe power that can be transferred from the heater plate to a liquid in the humidification chamber based, at least in part, on the calculated flow rate and the calculated maximum safe mass evaporation rate.

**In** a configuration, the heater plate temperature limit can be determined based, in part, on the calculated maximum safe temperature of the liquid in the humidification chamber and the calculated maximum safe power that can be transferred from the heater plate to the liquid in the humidification chamber.

**In** a configuration, the method further can comprise: calculating a maximum safe temperature of the liquid in the humidification chamber based, at least in part, on the calculated maximum safe mass evaporation rate; calculating a maximum safe power that can be transferred from the heater plate to the liquid in the humidification chamber based, at least in part, on the calculated maximum safe mass evaporation rate and the calculated flow rate; and calculating a heater plate temperature limit based, at least in part, on the calculated maximum safe temperature of the liquid in the humidification chamber and the calculated maximum safe power that can be transferred from the heater plate to the liquid in the humidification chamber.

**In** a configuration, the method further can comprise updating a current heater plate temperature limit with the calculated heater plate temperature limit.

**In** a configuration, the calculated flow rate can be a mass flow rate.

**In** a configuration, the calculated flow rate can be a volumetric flow rate.

**In** a configuration, the operating parameters can comprise sensor data and/or modelling parameters.

Disclosed, but not independently claimed, is a respiratory support device forming part of a respiratory support system which delivers a flow of gases to a patient, wherein the respiratory support device is configured to receive a humidification chamber containing a volume of liquid, and comprises: a heater plate configured to transfer heat to the humidification chamber, wherein an inlet of the humidification chamber is configured to receive the flow of gases to be heated and humidified, and an outlet of the humidification chamber is configured to be coupled to an inspiratory tube; a controller in electrical communication with the heater plate, wherein the controller is configured to control a heat output of the heater plate in order to adjust the heat transferred to the humidification chamber, wherein the controller is further configured to: calculate a flow rate of the flow of gases entering the humidification chamber based, at least in part, on operating parameters of the respiratory support device; calculate a maximum safe mass evaporation rate based, at least in part, on the calculated flow rate; and calculate a heater plate temperature limit based, at least in part, on the calculated maximum safe mass evaporation rate.

**In** a configuration, the controller can be further configured to determine a maximum power that may be provided to the heater plate based, at least in part, on the heater plate temperature limit and a current heater plate temperature.

**In** a configuration, the controller can be further configured to: compare the calculated heater plate temperature limit with a current heater plate temperature limit, and update the current maximum power that may be provided to the heater plate to be the determined maximum power that may be provided to the heater plate in response to the calculated heater plate temperature limit being lower than the current heater plate temperature limit.

**In** a configuration, the controller can be configured to disable power to the heater plate, and either disable or reduce power to the inspiratory tube (i.e. inspiratory tube heater) in response to the device failing to update the current maximum power that may be provided to the heater plate to be the calculated maximum power that may be provided to the heater plate, optionally within a threshold time.

**In** a configuration, the controller can be configured to disable power to the heater plate and/or disable power to the inspiratory tube (i.e. inspiratory tube heater) in response to the current heater plate temperature exceeding the calculated heater plate temperature limit by: 5°C for 10 minutes, or 10°C for 10 seconds, and/or in response to a current power supplied to the heater plate exceeding the determined maximum power that may be provided to the heater plate by a statistical variation based threshold (e.g., if the current power supplied to the heater plate exceeds the determined maximum power that may be supplied to the heater plate by 3 standard deviations).

**In** a configuration, the controller can be configured to reduce or disable power to a flow generator of the device in response to: the device failing to update the current maximum power that may be provided to the heater plate to be the calculated maximum power that may be provided to the heater plate, optionally within the threshold time; the current heater plate temperature exceeding the calculated heater plate temperature limit; and/or the current power supplied to the heater plate exceeding the determined maximum power that may be provided to the heater plate.

**In** a configuration, the controller can be further configured to maintain an existing maximum power that may be provided to the heater plate unless the calculated heater plate temperature limit is lower than the current heater plate temperature limit.

**In** a configuration, the controller can be further configured to increase the maximum power that may be provided to the heater plate in response to the calculated heater plate temperature limit being higher than the current heater plate temperature limit.

**In** a configuration, the current heater plate temperature limit can be a pre-set temperature limit.

**In** a configuration, the pre-set temperature limit can be a temperature above which a hardware safety feature is triggered.

**In** a configuration, the controller can be further configured to calculate a maximum safe temperature of the liquid in the humidification chamber based, at least in part, on the calculated flow rate.

**In** a configuration, the controller can be further configured to calculate a maximum safe power that can be transferred from the heater plate to the liquid in the humidification chamber based, at least in part, on the calculated flow rate and the calculated maximum safe mass evaporation rate.

In a configuration, the heater plate temperature limit can be determined based, in part, on the calculated maximum safe temperature of the liquid in the humidification chamber and a calculated maximum safe power that can be transferred from the heater plate to the liquid in the humidification chamber.

In a configuration, the controller can be further configured to: calculate a maximum safe temperature of the liquid in the humidification chamber based, at least in part, on the calculated maximum safe mass evaporation rate; calculate a maximum safe power that can be transferred from the heater plate to the liquid in the humidification chamber based, at least in part, on the calculated maximum safe mass evaporation rate and the estimated flow rate; and calculate a heater plate temperature limit based, at least in part, on the calculated maximum safe temperature of the liquid in the humidification chamber and the calculated maximum safe power that can be transferred from the heater plate to the liquid in the humidification chamber.

In a configuration, the controller can be further configured to update a current heater plate temperature limit with the calculated heater plate temperature limit.

In a configuration, the calculated flow rate can be a mass flow rate.

In a configuration, the calculated flow rate can be a volumetric flow rate.

In a configuration, the operating parameters can comprise sensor data and/or modelling parameters.

A respiratory support system can comprising: the respiratory support device of any of the configurations disclosed herein; and an inspiratory tube.

In a configuration, the controller can be configured to control a heat output to a heating element in the inspiratory tube
In a configuration, the respiratory support system further can comprise the humidification chamber.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
**Figure** 1 illustrates schematically a high flow respiratory system configured to provide a respiratory therapy to a patient.
**Figure** 2 is a front perspective view of an example high flow respiratory apparatus with a humidification chamber in position.
**Figure** 3 is a rear perspective view of the respiratory apparatus of Figure 2.
**Figure** 4 illustrates an example sensing chamber of the respiratory apparatus of Figure 2.
Figure 5 is a flow diagram of an illustrative process to reduce the risk that the enthalpy and/or the dew point of gases provided to a patient exceeds a respective predetermined threshold.
**Figure** 6 is a flow diagram of an illustrative process to calculate a maximum heater plate temperature for selected set points.

### DETAILED DESCRIPTION

Although certain examples are described below, those of skill in the art will appreciate that the disclosure extends beyond the specifically disclosed examples and/or uses and obvious modifications and equivalents thereof. Thus, it is intended that the scope of the disclosure herein disclosed should not be limited by any particular examples described below.

### Example Respiratory Support Systems

A respiratory support system can include a respiratory support device coupled to an inspiratory tube, which in turn can be coupled to a patient interface. The respiratory support device can include a humidifier and optionally also a flow generator. When the respiratory support device includes both the humidifier and the flow generator, the flow generator can either be enclosed in a housing separate from a housing of the humidifier, or be integrated with the humidifier into a single main device housing.

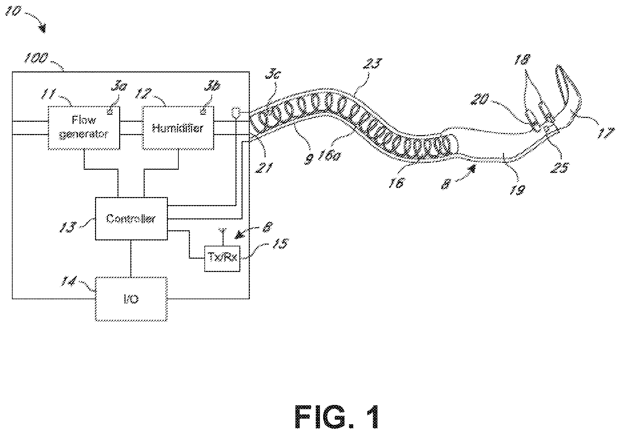

A schematic representation of a respiratory support system 10 is provided in Figure 1. The respiratory support system 10 can include a main device housing 100. The main device housing 100 can include a flow generator 11 that can be in the form of a motor/impeller arrangement, wherein the impeller is driven by the motor. The main device housing 100 can also include a humidifier 12. The humidifier 12 can include a receivable humidification chamber and a heater plate, for example. In the illustrated example, the humidification chamber may be positioned in contact with the heater plate (for example, such that the base of the chamber is in contact with the heater plate), and the heater plate may heat the contents of the humidification chamber to humidify gases passing from the flow generator to the humidifier. The main device housing 100 can also position a controller 13 and a user interface 14.

The user interface 14 can include a display and input device(s) such as button(s), a touch screen, a combination of a touch screen and button(s), or the like.

The controller 13 can include one or more hardware and/or software processors and can be configured or programmed to control the components of the system. For example, the controller can be configured to control the flow generator 11 to create a flow of gases for delivery to a patient, control the humidifier to humidify and/or heat the gases flow, receive user input from the user interface 14 for reconfiguration and/or user-defined operation of the respiratory support system 10, and output information (for example on the display) to the user. The user can be a patient, a healthcare professional, or another person.

With continued reference to Figure 1, an inspiratory tube 16 can be coupled (at one end) to a gases flow outlet 21 in the main device housing 100 and coupled (at the other end) to a patient interface 17. The patient interface 17 may be a non-sealing interface, such as a nasal cannula with a manifold 19 and nasal prongs 18. The gases flow outlet 21 may be on the humidifier 12. The inspiratory tube 16 can also be coupled to a face mask, a nasal mask, a nasal pillow mask, an endotracheal tube, a tracheostomy interface, or others. The inspiratory tube 16 may be part of a circuit through which a patient can be supplied a gases flow.

The gases flow can be generated by the flow generator 11, and humidified by the humidifier 12, before being delivered to the patient via the inspiratory tube 16 and the patient interface 17. The controller 13 can control the flow generator 11 to generate a gases flow at a desired flow rate. The controller can also control one or more valves to control mixing of air and oxygen (or other breathable gas). The controller 13 can control a heating element in the humidifier 12 (for example, a heating element in a heater plate of the humidifier) to heat the gases so that they are delivered to the patient at a desired temperature and/or humidity.

The inspiratory tube 16 can include a heating element 16a, such as a heater wire, to heat the gases as they flow through to the patient. The heating element 16a may extend through the length of the inspiratory tube 16 and may heat the gases to reduce the amount of condensation forming in the inspiratory tube 16. The heating element 16a can also be under the control of the controller 13. The heating element 16a is preferably embedded in the wall of the tube. Alternatively, the heating element 16a may be laid within the lumen of the tube.

The system 10 can use one or more sensors (that communicate with the controller 13) to monitor characteristics of the gases flow and/or operate the system 10 in a manner that provides suitable respiratory support. These sensors may include ultrasonic transducer(s), flow sensor(s) (such as thermistor flow sensors), pressure sensor(s), temperature sensor(s), humidity sensor(s), or other sensors. The gases flow characteristics can include gases concentration, flow rate, pressure, temperature, humidity, or others. The sensors 3a, 3b, 3c, 20, 25 can be placed in various locations in or on the main device housing 100, the inspiratory tube 16, and/or the patient interface 17. The sensors 3a, 3b, 3c, 20, 25 may also include an ambient temperature sensor. The ambient temperature sensor can be located anywhere where the ambient temperature sensor is exposed to the ambient environment. In one example, the ambient temperature sensor is positioned upstream of the flow generator. The ambient temperature sensor can be configured to measure the temperature of the incoming air from the atmosphere. The sensors 3a, 3b, 3c, 20, 25 may further include patient sensors to measure patient parameters (for example, blood oxygen saturation level (e.g. via a pulse oximeter), respiratory rate, body temperature, and the like). The sensors 3a, 3b, 3c, 20, 25 may be arranged in communication with the controller either by a wired or wireless configuration.

The controller 13 can receive output from the sensors to assist it in operating the respiratory support system 10 in a manner that provides suitable respiratory support. For example, the sensor outputs may assist the respiratory support system 10 to determine a suitable target temperature, flow rate, and/or pressure of the gases flow. Providing suitable respiratory support can include meeting, or exceeding, a patient's inspiratory demand.

The system 10 can include a wireless data transmitter and/or receiver, or a transceiver 15 to enable the controller 13 to receive data signals 8 in a wireless manner from the operation sensors and/or to control the various components of the system 10. Additionally, or alternatively, the data transmitter and/or receiver 15 can deliver data to a remote server or enable remote control of the system 10. The system 10 can include a wired connection, for example, using cables or wires, to enable the controller 13 to receive data signals 8 from the operation sensors and/or to control the various components of the system 10. In one example the data transmitted may comprise a modem that allows the apparatus to communicate with a remote system or a remote server to transmit data, e.g. therapy data or usage data. Additionally, the apparatus may also comprise other communication modules such as a Wi-Fi module and a Bluetooth Module. Further alarm information may be sent to a remote server (e.g. a remote therapy management system) along with other data such as therapy parameter and usage data. The remote therapy management system allows a clinician or other user to access information and generate reports that may be used to manage a patient's therapy.

In one example, the respiratory support system 10 comprises a high flow respiratory support apparatus. High flow respiratory support as discussed herein is intended to be given its typical ordinary meaning, as understood by a person of skill in the art. This generally refers to a respiratory support system delivering a targeted flow of humidified respiratory gases (via an intentionally unsealed patient interface) with flow rates generally intended to meet or exceed the patient's inspiratory demand. Typical patient interfaces include, but are not limited to, nasal or tracheal patient interfaces. In high flow therapy systems, patient interfaces may be unsealed. Typical flow rates for adults often range from, but are not limited to, about fifteen litres per minute to about sixty litres per minute or greater. Typical flow rates for paediatric patients (such as neonates, infants, and children) often range from, but are not limited to, about one litre per minute per kilogram of patient weight to about three litres per minute per kilogram of patient weight or greater. High flow respiratory support can also optionally include gas mixture compositions including supplemental oxygen and/or administration of therapeutic medicaments. High flow respiratory support is often referred to as nasal high flow (NHF), humidified high flow nasal cannula (HHFNC), high flow nasal oxygen (HFNO), high flow therapy (HFT), or tracheal high flow (THF), among other common names.

For example, in some configurations, for an adult patient 'high flow respiratory support' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow respiratory support' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow respiratory support apparatus with an adult patient, a neonatal patient, an infant patient, or a child patient, may deliver gases to the patient at a flow rate of between about 1 LPM and about 100 LPM, or at a flow rate in any of the sub-ranges outlined above.

The present system can also provide CPAP or bilevel therapies, among other respiratory therapies.

High flow respiratory support can be effective in meeting or exceeding the patient's inspiratory demand, increasing oxygenation of the patient's blood, and/or reducing the work of breathing. Additionally, high flow respiratory support may generate a flushing effect in the nasopharynx such that the anatomical dead space of the upper airways is flushed by the high incoming gases flow. The flushing effect can create a reservoir of fresh gases available for each breath, while minimising re-breathing of carbon dioxide and nitrogen, etc.

The patient interface for use in a high flow respiratory support can be a non-sealing interface to prevent barotrauma. Barotrauma can include tissue damage to the lungs or other organs of the patient's respiratory system due to differences in pressure relative to the atmosphere. The patient interface can be a nasal cannula with a manifold and nasal prongs, and/or a face mask, and/or a nasal pillows mask, and/or a nasal mask, and/or a tracheostomy interface, or any other suitable type of patient interface.

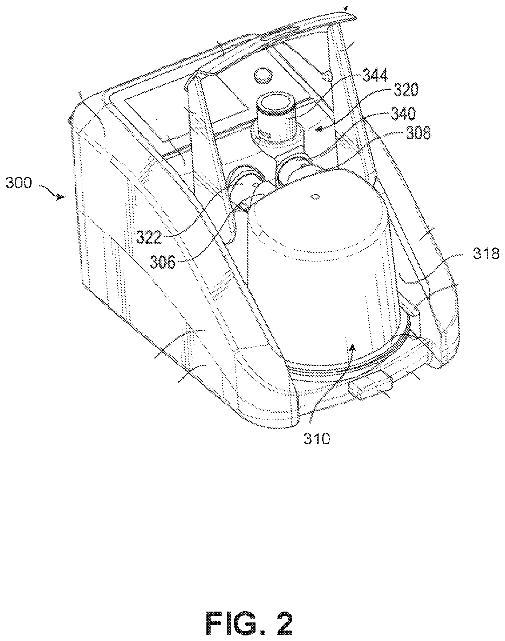

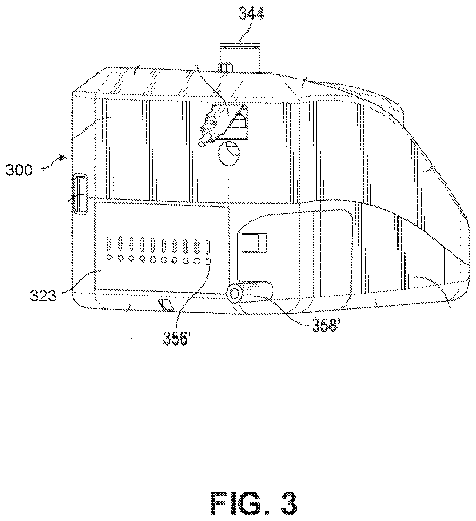

Figures 2 and 3 show an example respiratory support device of the respiratory support system 10. The device can include a housing 300, which encloses a flow generator. The flow generator may include a motor and sensor module. The motor and sensor module may be non-removable from the main housing 300. The motor and sensor module can also optionally be removable from the main housing 300. The housing 300 can also position a humidifier 318 for receipt of a removable humidification chamber 310. The removable humidification chamber 310 contains a suitable liquid such as water for heating and humidifying gases delivered to a patient. The humidification chamber 310 can be fluidly coupled to the device housing 300 with a linear slide-on motion into the humidifier 318. A gases outlet port 322 can establish a fluid communication between the motor and sensor module and an inlet 306 of the chamber 310.

Heated and humidified gases can exit an outlet 308 of the chamber 310 into a humidified gases return 340, which can include a removable L-shaped elbow. The removable elbow can further include a patient outlet port 344 for coupling to the inspiratory tube (such as the inspiratory tube 16 of Figure 1) to deliver gases to the patient interface 17. The gases outlet port 322, humidified gases return 340, and patient outlet port 344 can each have seals (such as O-ring seals or T-seals) to provide a sealed gases passageway between the device housing 300, the humidification chamber 310, and the inspiratory tube 16. A floor portion of the humidifier 318 in the housing 300 can include a heater arrangement such as a heater plate or other suitable heating element(s). This heater arrangement can heat the water in the humidification chamber 310 during a humidification process. Accordingly, the device in Figures 2 and 3 includes an integrated humidifier and flow generator in the housing 300.

As shown in Figure 3, the device can include an arrangement to enable the flow generator to deliver air, oxygen (or alternative auxiliary gas), or a suitable mixture thereof to the humidification chamber 310 and thereby to the patient. This arrangement can include an air inlet 356' in a rear wall 323 of the housing 300. The device can include a separate oxygen inlet port 358'. In the illustrated configuration, the oxygen inlet port 358' can be positioned adjacent one side of the housing 300 at a rear end thereof. The oxygen port 358' can be connected to an oxygen source such as a tank, or an oxygen blender. The oxygen inlet port 358' can be in fluid communication with a valve. The valve can suitably be a solenoid valve that enables the control of the amount of oxygen that is added to the gases flow that is delivered to the humidification chamber 310.

The housing 300 can position suitable electronics boards, such as sensing circuit boards. The electronics boards can contain, or can be in electrical communication with, suitable electrical or electronics components, such as but not limited to microprocessors, capacitors, resistors, diodes, operational amplifiers, comparators, and switches. One or more sensors can be used with the electronic boards. Components of the electronics boards (such as but not limited to one or more microprocessors) can act as the controller 13 of the apparatus. One or more of the electronics boards can be in electrical communication with the electrical components of the system 10, including but not limited to the display unit and user interface 14, motor, valve, and the heater plate and/or the heated inspiratory tube to provide the desired flow rate of gases, humidify and heat the gases flow to appropriate levels, and supply appropriate quantities of oxygen (or quantities of an alternative auxiliary gas) to the gases flow.

As mentioned above, operation sensors, such as flow, temperature, humidity, and/or pressure sensors can be placed in various locations in or on the respiratory support device, the inspiratory tube 16, and/or the patient interface 17. The electronics boards can be in electrical communication with those sensors. Output from the sensors can be received by the controller 13, to assist the controller 13 to operate the respiratory support system 10 in a manner that provides optimal respiratory support, including meeting inspiratory demand.

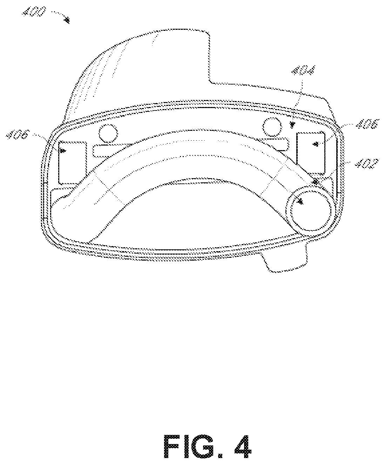

As shown in Figure 4, the mixed air can exit the flow generator and enter a flow path 402 in a sensor chamber 400, which can be located in the motor and sensor module. A sensing circuit board 404 with sensors, such as ultrasonic sensors 406 and/or heated thermistor flow sensors, can be positioned in the sensor chamber 400 such that the sensing circuit board is at least partially immersed in the gases flow. At least some of the sensors on the sensing circuit board can be positioned within the gases flow to measure gas properties within the flow. After passing through the flow path 402 in the sensor chamber 400, the gases can exit to the humidification chamber 310.

Immersing at least part of the sensing circuit board and sensors in the flow path can increase the accuracy of measurements because (relative to sensors that are not immersed) sensors that are immersed in the flow are more likely to be subject to the same conditions, such as temperature and pressure, as the gases flow. Therefore, these immersed sensors may provide a better representation of the gases flow characteristics.

As shown in Figure 4, the flow path 402 can have a curved shape. The sensing circuit board 404 can include sensors such as acoustic transmitters and/or receivers, humidity sensors, temperature sensors, thermistors, and the like. A gases flow rate may be measured using at least two different types of sensors. The first type of sensor can include a thermistor, which can determine a flow rate by monitoring heat transfer between the gases flow and the thermistor. The thermistor flow sensor can run the thermistor at a constant target temperature within the flow when the gases flow around and past the thermistor. The sensor can measure an amount of power required to maintain the thermistor at the target temperature. The target temperature can be configured to be higher than a temperature of the gases flow, such that more power is required to maintain the thermistor at the target temperature at a higher flow rate.

The thermistor flow rate sensor can also maintain a plurality of (for example, two, three, or more) constant temperatures on a thermistor to avoid the difference between the target temperature and the gases flow temperature from being too small or too large. The plurality of different target temperatures can allow the thermistor flow rate sensor to be accurate across a large temperature range of the gas. For example, the thermistor circuit can be configured to be able to switch between two different target temperatures, such that the temperature of the gases flow can always fall within a certain range relative to one of the two target temperatures (for example, not too close and not too far). The thermistor circuit can be configured to operate at a first target temperature of about 50 ⁰C to about 70 ⁰C, or about 66 °C. The first target temperature can be associated with a desirable flow temperature range of between about 0 ⁰C to about 60 ⁰C, or about 0 °C to about 40 °C. The thermistor circuit can be configured to operate at a second target temperature of about 90 ⁰C to about 110 ⁰C, or about 100 °C. The second target temperature can be associated with a desirable flow temperature range of between about 20 ⁰C to about 100 ⁰C, or about 30 °C and about 70 °C.

The controller can be configured to adjust the thermistor circuit to change between at least the first and second target temperature modes by connecting or bypassing a resistor within the thermistor circuit. The thermistor circuit can be arranged as a Wheatstone bridge configuration comprising a first voltage divider arm and a second voltage divider arm. The thermistor can be located on one of the voltage divider arms.

The second type of sensor can include an acoustic (such as ultrasonic) sensor assembly. Acoustic sensors (including acoustic transmitters and/or receivers) can be used to measure a time of flight of acoustic signals to determine gas velocity and/or composition. The controller 13 can receive the output from acoustic sensor(s) to assist it in operating the respiratory support system 10 in a manner that provides suitable respiratory support. In one ultrasonic sensing topology (including ultrasonic transmitters and/or receivers), a driver causes a first sensor, such as an ultrasonic transducer, to produce an ultrasonic pulse in a first direction. A second sensor, such as a second ultrasonic transducer, receives this pulse and provides a measurement of the time of flight of the pulse between the first and second ultrasonic transducers. Using this time of flight measurement, the speed of sound of the gases flow between the ultrasonic transducers can be calculated by a processor or controller of the respiratory apparatus. The second sensor can also transmit, and the first sensor can receive a pulse in a second direction opposite the first direction to provide a second measurement of the time of flight, allowing characteristics of the gases flow, such as a flow rate or velocity, to be determined. In another acoustic sensing topology, acoustic pulses transmitted by an acoustic transmitter, such as an ultrasonic transducer, can be received by acoustic receivers, such as microphones. The acoustic pulses can be transmitted along the flow path of the gases, thereby allowing the acoustic sensors to be used to measure a flow rate or velocity of the gases.

Readings from both the first and second types of sensors can be combined to determine a more accurate flow measurement. For example, a previously determined flow rate and one or more outputs from one of the types of sensors can be used to determine a predicted current flow rate. The predicted current flow rate can then be updated using one or more outputs from the other one of the first and second types of sensors, in order to calculate a final flow rate.

Further operation sensors can include one or more heater plate temperature sensors located adjacent to or at (for example, immediately below) a top plate of the heater plate. The heater plate temperature sensor can be configured to measure a temperature of the heater plate.

Various temperature sensors can also be included throughout the respiratory support system. An ambient temperature sensor can be located anywhere where the ambient temperature sensor is exposed to the ambient environment. The ambient temperature sensor can be configured to measure the temperature of the incoming air from the atmosphere. An outlet temperature sensor can optionally be located at or near a humidification chamber outlet, or at a chamber end (opposite the patient end) of the inspiratory tube. The outlet temperature sensor can be configured to measure a temperature of the gases stream exiting the humidification chamber. A patient-end temperature sensor can optionally be located at the patient end of the inspiratory tube. The patient end temperature sensor can also optionally be located in or on the patient interface. The patient end temperature sensor can be in communication with the controller using, for example, wired or wireless communication.

The respiratory support system can optionally include a flow sensor configured to measure the gases flow through the system. The flow sensor can be located at or near the humidification chamber outlet, at or near the chamber end of the inspiratory tube, and/or adjacent to the outlet temperature sensor at or near the humidification chamber outlet. The system can optionally include both temperature and flow sensors at or near the chamber end of the inspiratory tube. These sensors may be in communication with one or more of the controllers using, for example, wired or wireless communication. The controllers can also optionally be in communication with one or more other sensors, which can measure the humidity, temperature, pressure, flow, and/or other characteristics of the gases flow.

In the respiratory support system described above, a temperature probe can also optionally be placed within the volume of water in the humidification chamber. Additionally, and/or alternatively, contactless temperature sensors (such as infrared sensors) can be used to measure a temperature of the heater plate, and/or a temperature of the contents of the humidification chamber, and/or a temperature of the gases path.

### Example Methods for Reducing the Risk that the Enthalpy and/or Dew Point of Gases at the Patient End Exceeds a Threshold

Protection against a condition in which the enthalpy and/or the dew point of the gases leaving the inspiratory tube exceeds a threshold can be incorporated in any of the respiratory support systems disclosed herein. Such a condition may occur when a dew point of the heated gases at the patient end of the inspiratory tube exceeds a predetermined threshold (for example, a dew point of from about 37°C to about 47°C, or from about 40°C to about 45°C, or at about 43°C).

When using a respiratory support device, protecting against the enthalpy and/or dew point of the gases causing harm or discomfort may be balanced against providing effective patient treatment. The technology used in current respiratory support systems may not always achieve this balance. For example, current respiratory support systems may apply a heater plate temperature limit to the heater plate of the respiratory support device such that the heater plate temperature never exceeds a maximum heater plate temperature. While setting a static heater plate temperature limit may appropriately limit the dew point temperature at the patient interface during operation with high temperature and flow set points, it may allow an excessively high dew point temperature at the patient interface during operation with a high temperature set point and a low flow set point. Conversely, a heater plate temperature limit that appropriately limits the dew point temperature at the patient interface during operation with low temperature and flow set points, may prevent the device from achieving high temperature and flow set points.

Achieving a balance, between providing protection against the enthalpy and/or dew point of the gases causing harm or discomfort and providing effective patient treatment, is especially relevant for high flow therapy (the respiratory support system described herein may be configured to provide high flow therapy). High flow therapy requires high levels of humidity for patient comfort and to ensure patient airway moisture is maintained. The high levels of humidity improve patient usage i.e. patient compliance to the therapy for extended periods of time. High humidity improves comfort and allows a patient to use the high flow therapy device (i.e. the respiratory support system) for extended periods of time. The high humidity also maintains the function of the patient's natural mucociliary transport within the patient's lungs and airways to move mucous and improve breathing. The temperature of the water and gases used in the therapy must therefore be carefully maintained to ensure patient safety. Further, high flow devices may have a wide range of selectable set points. This means that there may be a wide range of operating parameters at which effective patient treatment needs to be provided. For example, a high flow device may produce flow rates between 0 L/min to 100 L/min or more. The respiratory support system disclosed herein may maintain the balance between patient safety and effective treatment across various therapy parameters.

With examples of the respiratory support system disclosed herein, a controller of a respiratory device (for example, a controller that controls the humidifier, blower, or both) can dynamically calculate a maximum heater plate power limit with which to operate the heater plate. The maximum heater plate power limit may be based at least in part on user input. The maximum heater plate power limit may be calculated such that the enthalpy and/or dew point of the gases do not exceed a predetermined threshold. The controller can dynamically calculate the maximum heater plate power limit by continuously calculating and updating the maximum heater plate power limit to accommodate conditions of the respiratory support system in real time. The controller can receive measurements from real-time sensors in the respiratory support system and, using the measurements from the sensors, calculate a dynamic heater plate temperature limit. The heater plate temperature may be maintained below the heater plate temperature limit throughout a respiratory support session. In some arrangements, the controller can calculate a maximum heater plate power limit to deliver to the heater plate, where the maximum heater plate power limit prevents the heater plate temperature from exceeding the heater plate temperature limit. The heater plate power can be controlled by adjusting the voltage, current, or a combination of both.

In other words, the controller can adjust the heater plate power limit to accommodate conditions of the respiratory support system in real time. The controller can limit the heater plate temperature such that the enthalpy and/or dew point of the gases are unlikely to exceed the predetermined threshold (for example, such that the enthalpy and/or dew point of the gases are less than or equal to the predetermined threshold). The controller can do this by calculating a maximum heater plate power limit based on real time sensor data and provide power to the heater plate within this limit.

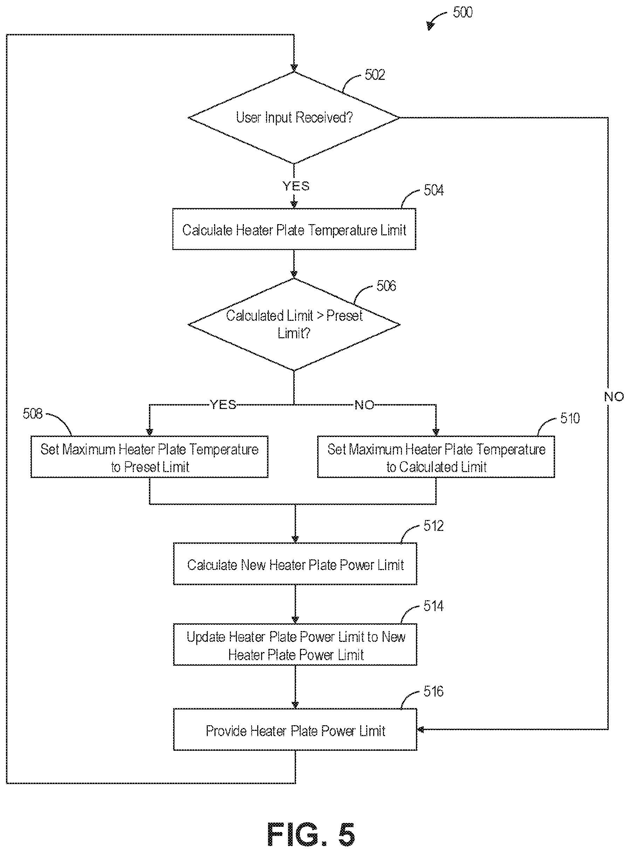

Figure 5 illustrates a process by which the controller may reduce the risk that the enthalpy and/or the dew point of gases provided to a patient exceeds a respective predetermined threshold. The controller can set and update respiratory support system operational limits when the controller receives certain user inputs (for example, via the user interface described above). Specifically, in response to user input and/or real-time data received from device sensors, the controller can determine one or more control outputs, which can send signals to adjust the energy delivered to the heater plate. User inputs can include, for example, when the user starts a new respiratory support session, changes to the setting on the respiratory device during a current session, or otherwise.

The process 500 may begin at decision block 502, where the controller can determine whether a user input has been received. If a user input is not received, the controller can maintain the current power limit (configured to provide the desired respiratory support to the patient) to the heater plate and continue the respiratory support session. This case, in which a user input is not received, is represented by the direct path from block 502 to block 516. The user input may be one or more of: a command to initiate a new respiratory support session, changes to one or more device set points, or the like.

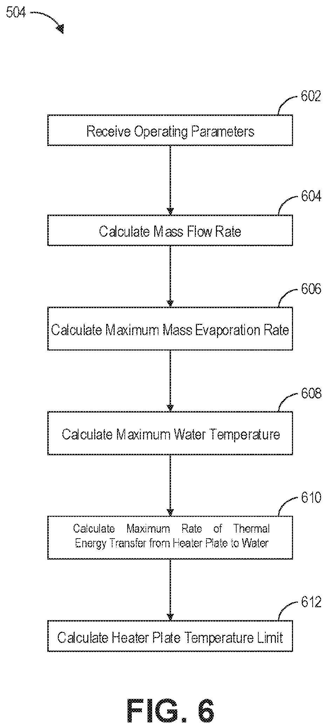

If a user input is received, the controller can proceed to block 504 to calculate a heater plate temperature limit that reduces the likelihood that the enthalpy and/or the dew point of gases provided to a patient exceeds a respective predetermined threshold. The heater plate temperature limit can be based on current device parameters as determined, in part, by sensor data in real time. Further disclosure related to calculating the heater plate temperature limit is described herein, in relation to Figure 6. The process 500 can then proceed to block 506.

At decision block 506, the controller can compare the heater plate temperature limit calculated in block 504 with a preset heater plate temperature limit. The preset heater plate temperature limit can be a temperature above which the hardware safety system will activate. The hardware safety system may include a switch that opens when the measured heater plate temperature exceeds the preset heater plate temperate limit, thereby discontinuing power delivery to the heater plate. The heater plate thus may not exceed the preset heater plate temperature limit to reduce likelihood of harm to the patient. The heater plate may be reset by power cycling the respiratory support device to reset the tripped switch.

If the calculated heater plate temperature limit is greater than the preset heater plate temperature limit, the process can proceed to block 508 and the controller can set the maximum heater plate temperature limit to be equal to the preset heater plate temperate limit. If the calculated heater plate temperature limit is less than the preset heater plate temperature limit, the process can proceed to block 510. At block 510, the controller can set the maximum heater plate temperature limit to be equal to the calculated heater plate temperature limit. In other words, the maximum heater plate temperature is the lesser of the calculated heater plate temperature limit and the preset heater plate temperature limit. In other words, the maximum heater plate temperature limit is set such that the heater plate cannot exceed the hardware safety system temperature limit.

At block 512, the controller can calculate a new heater plate power limit based on current device parameters. For example, the new heater plate power limit can be based on, at least, the maximum heater plate temperature and a current, measured heater plate temperature. In another example, the new heater plate power limit may be based on, at least, a change in ambient temperature. At block 514, the controller can update the heater plate power limit to be the new heater plate power limit calculated in block 512. At block 516, the controller can provide the updated power limit to the heater plate. The controller can maintain the heater plate power limit at the limits calculated in the process 500. The process 500 can thus repeat as the controller maintains the heater temperature or power limit while continuously checking for user input at block 502.

By controlling the heater plate power limit as a function of real-time sensor data, the controller can dynamically control the maximum amount of heat that may be delivered to the water, and, therefore, the maximum temperature of the water in the humidification chamber. The controller can thus control the humidification of the flow of gases provided to the patient during a respiratory support session. In this way, the controller can reduce the risk that the enthalpy and/or the dew point of the gases provided to the patient will exceed respective predetermined thresholds, without unduly restricting the range of achievable heater plate temperatures and device set points.

In some situations the new maximum heater plate power limit may not have been implemented or may have been implemented too slowly as a result of, for example, a respiratory support system malfunction that had an impact on the heater plate control or other related hardware. The controller may implement safety responses in those situations. The controller can determine whether the calculated maximum heater plate power limit is implemented by the respiratory support system. If the respiratory device has failed to update the current maximum power limit to the heater plate to be the calculated maximum power limit to the heater plate (for example, after a certain threshold time), the controller can cut (i.e. disable) power to the heater plate, and can either disable or reduce power to the inspiratory tube (i.e. to the inspiratory tube heater). The threshold time may be for example, one second, five seconds, fifteen seconds, 30 seconds, one minute, two minutes, five minutes, or otherwise. Alternatively or additionally, the controller can also determine whether the current heater plate temperature exceeds the determined heater plate temperature limit and/or whether the current power supplied to the heater plate exceeds the determined maximum power limit to the heater plate. The controller can determine how much and/or for how long the determined heater plate temperature limit and/or the determined maximum power limit to the heater plate has been exceeded. For example, the controller can cut (i.e. disable) power to the heater plate and/or cut power to the inspiratory tube (i.e. to the inspiratory tube heater) if: the current heater plate temperature exceeds the determined heater plate temperature limit by 5°C for 10 minutes, or 10°C for 10 seconds; and/or a current power supplied to the heater plate exceeds the determined maximum power limit to the heater plate by a statistical variation based threshold (e.g., if the current power supplied to the heater plate exceeds the determined maximum power limit to the heater plate by 3 standard deviations). Alternatively or additionally , the controller can reduce or cut (i.e. disable) power to the flow generator in response to: the respiratory device failing to update the current maximum power limit to the heater plate to be the calculated maximum power limit to the heater plate, optionally within the threshold time disclosed herein; the current heater plate temperature exceeding the determined heater plate temperature limit; and/or the current power supplied to the heater plate exceeding the determined maximum power limit to the heater plate.

Figure 6 is a flow diagram of an illustrative process to calculate a maximum heater plate temperature for selected set points, as shown in block 504 of Figure 5. The process 504 can begin at block 602 when the controller receives current operating parameters. Current operating parameters can be real-time data from sensors throughout the respiratory support system. Current operating parameters can also include parameters defined for the current respiratory therapy session via user inputs. Current operating parameters may include, but are not limited to, ambient temperature, patient blood oxygen saturation level, humidification chamber water temperature, and gases flow rate.

At block 604, the controller can optionally calculate the mass flow rate of gases entering the humidification chamber based on the current operating parameters. At block 606, the controller can optionally calculate a maximum mass evaporation rate for the current operating parameters, based at least on the calculated mass flow rate. The maximum mass evaporation rate can be a maximum safe mass evaporation rate, which is an evaporation rate under which the subsequent flow of gases at the patient interface will not cause harm or discomfort to the patient. The maximum mass evaporation rate can be a dynamic limit which can be updated by the controller as operating parameters change.

At block 608, the controller can calculate a maximum water temperature for the water in the humidification chamber, based on the current operating parameters (and optionally based on the calculated maximum mass evaporation rate). The maximum water temperature can be a maximum safe water temperature, which is a temperature at which the water in the humidification chamber can be maintained without the subsequent flow of gases at the patient interface harming or causing discomfort to the patient. The maximum water temperature can be a dynamic limit which can be updated by the controller as operating parameters change.

At block 610, the controller can calculate a maximum rate of thermal energy to be transferred from the heater plate to the water in the humidification chamber. The maximum thermal energy transfer rate can be a limit on heat transfer from the heater plate to the water in the humidification chamber, such that the water remains at or below the maximum water temperature calculated at block 608. The maximum thermal energy transfer rate may be a maximum safe power, which is a rate of thermal energy transfer to the water under which the subsequent flow of gases at the patient interface will not cause harm or discomfort to the patient. The maximum thermal energy transfer rate can be based at least on the calculated mass flow rate and maximum mass evaporation rate. The maximum thermal energy transfer rate can be a dynamic limit which can be updated by the controller as operating parameters change.

At block 612, the controller can calculate the heater plate temperature limit. The heater plate temperature limit can be based at least on the maximum power transfer. The heater plate temperature limit can be a dynamic limit which can be updated by the controller as operating parameters change. The heater plate temperature limit can be used to calculate a heater plate power limit, as described in Figure 5. The heater plate temperature limit can be the input to block 506 in the process illustrated by Figure 5.

The foregoing methods may be implemented by any respiratory support system which provides humidified gases in respiratory support, including, but not limited to, bi-level pressure respiratory support, CPAP respiratory support, or high flow nasal cannula respiratory support.

### Terminology

Although this disclosure has been described in the context of certain embodiments and examples, it will be understood by those skilled in the art that the disclosure extends beyond the specifically disclosed embodiments to other alternative embodiments and/or uses and obvious modifications and equivalents thereof. In addition, while several variations of the embodiments of the disclosure have been shown and described in detail, other modifications, which are within the scope of this disclosure, will be readily apparent to those of skill in the art. It is also contemplated that various combinations or sub-combinations of the specific features and aspects of the embodiments may be made and still fall within the scope of the disclosure. For example, features described above in connection with one embodiment can be used with a different embodiment described herein and the combination still fall within the scope of the disclosure. It should be understood that various features and aspects of the disclosed embodiments can be combined with, or substituted for, one another in order to form varying modes of the embodiments of the disclosure. Thus, it is intended that the scope of the disclosure herein should not be limited by the particular embodiments described above. Accordingly, unless otherwise stated, or unless clearly incompatible, each embodiment of this invention may comprise, additional to its essential features described herein, one or more features as described herein from each other embodiment of the invention disclosed herein.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments.

Furthermore, certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as a subcombination or variation of a subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognise that the disclosure may be embodied or carried out in a manner that achieves one advantage or a group of advantages as taught herein without necessarily achieving other advantages as may be taught or suggested herein.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without other input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, 0.1 degree, or otherwise.

Any methods disclosed herein need not be performed in the order recited. The methods disclosed herein include certain actions taken by a practitioner; however, they can also include any third-party instruction of those actions, either expressly or by implication. For example, actions such as "controlling a motor speed" include "instructing controlling of a motor speed."

All of the methods and tasks described herein may be performed and fully automated by a computer system. The computer system may, in some cases, include multiple distinct computers or computing devices (e.g., physical servers, workstations, storage arrays, cloud computing resources, etc.) that communicate and interoperate over a network to perform the described functions. Each such computing device typically includes a processor (or multiple processors) that executes program instructions or modules stored in a memory or other non-transitory computer-readable storage medium or device (e.g., solid state storage devices, disk drives, etc.). The various functions disclosed herein may be embodied in such program instructions, and/or may be implemented in application-specific circuitry (e.g., ASICs or FPGAs) of the computer system. Where the computer system includes multiple computing devices, these devices may, but need not, be co-located. The results of the disclosed methods and tasks may be persistently stored by transforming physical storage devices, such as solid state memory chips and/or magnetic disks, into a different state. In some embodiments, the computer system may be a cloud-based computing system whose processing resources are shared by multiple distinct business entities or other users.

The scope of the present disclosure is not intended to be limited by the specific disclosures of preferred embodiments in this section or elsewhere in this specification. The language of the claims is to be interpreted broadly based on the language employed in the claims and not limited to the examples described in the present specification or during the prosecution of the application, which examples are to be construed as non-exclusive. The invention is defined by the claims which follow.

## Claims

1. A respiratory support device forming part of a respiratory support system (10) which delivers a flow of gases to a patient, wherein the respiratory support device is configured to receive a humidification chamber (310) containing a volume of liquid, the respiratory support device comprising:
a heater plate configured to transfer heat to the humidification chamber (310), wherein an inlet (306) of the humidification chamber (310) is configured to receive the flow of gases to be heated and humidified, and an outlet (308) of the humidification chamber (310) is configured to be coupled to an inspiratory tube (16);
a first sensor configured to measure a current temperature of the heater plate;
at least one or more other sensors configured to measure parameters relating to at least one of the gases flowing through the device, an ambient environment, or hardware within the device, wherein each of the at least one or more other sensors is configured to measure a different parameter than the rest of the at least one or more other sensors;
a user interface (14) configured to receive user input; and
a controller (13) in electrical communication with the heater plate, the first and at least one or more other sensors, and the user interface (14), wherein the controller (13) is configured to control a heat output of the heater plate in order to adjust the heat transferred to the humidification chamber (310), wherein the controller (13) is further configured to:
determine a heater plate temperature limit (504) in response to the user input received via the user interface (14) and based, at least in part, on the parameters measured by the at least one or more other sensors; and
calculate a maximum power limit (512) to the heater plate based, at least in part, on the heater plate temperature limit and data from the first sensor, wherein the maximum heater plate power limit (512) prevents the heater plate temperature from exceeding the heater plate temperature limit (504).

2. The device of Claim 1, wherein the heater plate temperature limit is a temperature at which the heater plate is configured to operate such that a dew point of the gases is below a limit that can result in injury to the patient.

3. The device of claim 1 or claim 2, wherein the user input comprises at least one of: a temperature set point change, a dew point set point change, a flow rate set point change, or any combination thereof.

4. The device of any one of claims 1-3, wherein the controller (13) is configured to update a current maximum power limit to the heater plate to be the calculated maximum power limit to the heater plate in response to the determined heater plate temperature limit being lower than a current heater plate temperature limit, and/or optionally wherein the controller (13) is configured to maintain an existing maximum power limit to the heater plate unless the determined heater plate temperature limit is lower than the current heater plate temperature limit, and/or optionally wherein the current heater plate temperature limit is a pre-set temperature limit, and optionally wherein the pre-set temperature limit is a temperature limit above which a hardware safety feature is triggered.

5. The device of any one of claims 1-4, wherein the power provided to the heater plate is dependent on a duty cycle.

6. The device of any one of claims 1-5, wherein the outlet of the humidification chamber (310) comprises a removable outlet elbow including a patient outlet port (344) for coupling to the inspiratory tube (16), and optionally wherein the at least one or more other sensors comprises two or more other sensors.

7. A respiratory support system (10) comprising:
the respiratory support device of any one of claims 1-6; and
the inspiratory tube (16).

8. The system of claim 7, wherein the parameters measured by the at least one or more other sensors comprise at least one of: a flow rate of the gases within a flow path of the system, a temperature measured at the outlet of the humidification chamber, a dew point measured at the outlet (308) of the humidification chamber (310), a temperature measured at the inlet of the humidification chamber (310), a dew point measured at the inlet of the humidification chamber (310), an ambient temperature, a pressure of the gases within the flow path of the system, or an electrical power, current, or voltage supplied to the heater plate, and optionally wherein the flow rate of the gases is a mass flow rate, or optionally wherein the flow rate of the gases is a volumetric flow rate.

9. The system of any one of claim 7 or claim 8, wherein the inspiratory tube (16) is a heated inspiratory tube, and wherein the controller (13) is configured to control a heat output to a heating element (16a) in the inspiratory tube (16).

10. The system of any one of claims 7-9, further comprising the humidification chamber (310), and/or optionally further comprising an unsealed patient interface (17), and/or optionally further comprising a flow generator (11) optionally located in a housing (100) of the device, and optionally wherein the flow generator (11) comprises a blower.

11. The system of any one of claims 7-10, further comprising at least one of a temperature sensor or dew point sensor at or near a patient end of the inspiratory tube (16).

12. A method of controlling a respiratory support device, wherein the respiratory support device forms part of a respiratory support system (10) and is configured to receive a humidification chamber (310) to heat and humidify the flow of gases, the method comprising:
under control of a controller (13) of the respiratory support device,
receiving data from a first sensor and at least two other sensors, the first sensor configured to measure a current temperature of a heater plate of the respiratory support device and the at least two other sensors configured to measure parameters relating to at least one of the gases flowing through the device, an ambient environment, or hardware within the device, wherein each of the at least two other sensors is configured to measure a different parameter than the rest of the at least two or more sensors;
receiving user input, via a user interface (14), wherein the user input changes at least one parameter setting for the respiratory support device;
determining a heater plate temperature limit in response to the user input received via the user interface (14) and based, at least in part, on data from the at least two other sensors; and
calculating a maximum power limit to the heater plate based, at least in part, on the heater plate temperature limit and the data from the first sensor, wherein the maximum heater plate power limit prevents the heater plate temperature from exceeding the heater plate temperature limit.

13. The method of claim 12, wherein the power provided to the heater plate is dependent on a duty cycle, and/or optionally wherein the heater plate temperature limit is a temperature at which the heater plate is configured to operate without a dew-point temperature of the gases directed into the user's airway exceeding a value that can result in injury to the patient, and/or optionally wherein the user input comprises at least one of: a temperature set point change, a dew point set point change, or a flow rate set point change.

14. The method of claim 12 or claim 13, further comprising updating a current maximum power limit to the heater plate to be the calculated maximum power limit to the heater plate in response to the determined heater plate temperature limit being lower than a current heater plate temperature limit, and/or optionally further comprising maintaining an existing maximum power limit to the heater plate unless the determined heater plate temperature limit is lower than the current heater plate temperature limit, and/or optionally wherein the current heater plate temperature limit is a pre-set temperature limit, and optionally wherein the pre-set temperature limit is a temperature limit above which a hardware safety feature is triggered.

15. The method of any one of claims 12-14, wherein the parameters measured by the at least two other sensors comprise at least two of: a flow rate of the gases within a flow path of the respiratory support system, a temperature measured at an outlet of the humidification chamber (310), a temperature measured at the inlet of the humidification chamber (310), an ambient temperature, a pressure of the gases within the flow path of the system, or an electrical power, current, or voltage supplied to the heater plate, and optionally wherein the flow rate of the gases is a mass flow rate, or optionally wherein the flow rate of the gases is a volumetric flow rate.

## Patentansprüche

1. Atemunterstützungsvorrichtung, die einen Teil eines Atemunterstützungssystems (10) bildet, das einem Patienten einen Strom von Gas zuführt, wobei die Atemunterstützungsvorrichtung konfiguriert ist, um eine Befeuchtungskammer (310) aufzunehmen, die ein Flüssigkeitsvolumen enthält, die Atemunterstützungsvorrichtung umfassend:
eine Heizplatte, die konfiguriert ist, um Wärme auf die Befeuchtungskammer (310) zu übertragen, wobei ein Einlass (306) der Befeuchtungskammer (310) konfiguriert ist, um den Strom von zu erwärmenden und zu befeuchtenden Gasen aufzunehmen, und ein Auslass (308) der Befeuchtungskammer (310) konfiguriert ist, um an einen Inspirationsschlauch (16) gekoppelt zu werden;
einen ersten Sensor, der konfiguriert ist, um eine aktuelle Temperatur der Heizplatte zu messen;
mindestens einen oder mehrere andere Sensoren, die konfiguriert sind, um Parameter zu messen, die sich auf mindestens eines der Gase, die durch die Vorrichtung strömen, eine Umgebung oder Hardware innerhalb der Vorrichtung beziehen, wobei jeder des mindestens einen oder der mehreren anderen Sensoren konfiguriert ist, um einen unterschiedlichen Parameter als im Übrigen des mindestens einen oder der mehreren anderen Sensoren zu messen;
eine Benutzerschnittstelle (14), die konfiguriert ist, um Benutzereingaben zu empfangen; und
eine Steuerung (13) in elektrischer Kommunikation mit der Heizplatte, dem ersten und mindestens einem oder mehreren anderen Sensoren und der Benutzerschnittstelle (14), wobei die Steuerung (13) konfiguriert ist, um eine Wärmeleistung der Heizplatte zu steuern, um die Wärme anzupassen, die auf die Befeuchtungskammer (310) übertragen wird, wobei die Steuerung (13) ferner konfiguriert ist zum:
Bestimmen einer Heizplattentemperaturgrenze (504) als Reaktion auf die Benutzereingabe, die über die Benutzerschnittstelle (14) empfangen wird und, mindestens teilweise basierend, auf den Parametern, die durch den mindestens einen oder die mehreren anderen Sensoren gemessen werden; und
Berechnen einer maximalen Leistungsgrenze (512) für die Heizplatte basierend mindestens teilweise auf der Heizplattentemperaturgrenze und Daten von dem ersten Sensor, wobei die maximale Heizplattenleistungsgrenze (512) verhindert, dass die Heizplattentemperatur die Heizplattentemperaturgrenze (504) überschreitet.

2. Vorrichtung nach Anspruch 1, wobei die Heizplattentemperaturgrenze eine Temperatur ist, bei der die Heizplatte konfiguriert ist, um derart betrieben zu werden, dass ein Taupunkt der Gase unter einer Grenze liegt, der zu einer Verletzung des Patienten führen kann.

3. Vorrichtung nach Anspruch 1 oder 2, wobei die Benutzereingabe mindestens eines umfasst von: einer Temperatursollwertänderung, einer Taupunktsollwertänderung, einer Strömungsgeschwindigkeitssollwertänderung oder einer beliebigen Kombination davon.

4. Vorrichtung nach einem der Ansprüche 1 bis 3, wobei die Steuerung (13) konfiguriert ist, um eine aktuelle maximale Leistungsgrenze für die Heizplatte auf die berechnete maximale Leistungsgrenze für die Heizplatte zu aktualisieren, als Reaktion darauf, dass die bestimmte Heizplattentemperaturgrenze niedriger als eine aktuelle Heizplattentemperaturgrenze ist, und/oder optional wobei die Steuerung (13) konfiguriert ist, um eine bestehende maximale Leistungsgrenze für die Heizplatte beizubehalten, es sei denn, die bestimmte Heizplattentemperaturgrenze niedriger als die aktuelle Heizplattentemperaturgrenze ist, und/oder optional wobei die aktuelle Heizplattentemperaturgrenze eine voreingestellte Temperaturgrenze ist, und optional wobei die voreingestellte Temperaturgrenze eine Temperaturgrenze ist, oberhalb derer ein Hardware-Sicherheitsmerkmal ausgelöst wird.

5. Vorrichtung nach einem der Ansprüche 1 bis 4, wobei die Leistung, die der Heizplatte bereitgestellt wird, von einem Tastverhältnis abhängig ist.

6. Vorrichtung nach einem der Ansprüche 1 bis 5, wobei der Auslass der Befeuchtungskammer (310) einen abnehmbaren Auslasswinkel einschließlich eines Patientenauslassanschlusses (344) zum Koppeln an den Inspirationsschlauch (16) umfasst, und optional wobei der mindestens eine oder die mehreren anderen Sensoren zwei oder mehr andere Sensoren umfassen.

7. Atemunterstützungssystem (10), umfassend:
die Atemunterstützungsvorrichtung nach einem der Ansprüche 1 bis 6; und
der Inspirationsschlauch (16).

8. System nach Anspruch 7, wobei die Parameter, die durch den mindestens einen oder die mehreren anderen Sensoren gemessen werden, mindestens eines umfassen von: einer Strömungsgeschwindigkeit der Gase innerhalb eines Strömungswegs des Systems, einer Temperatur, die an dem Auslass der Befeuchtungskammer gemessen wird, einem Taupunkt, der an dem Auslass (308) der Befeuchtungskammer (310) gemessen wird, einer Temperatur, die an dem Einlass der Befeuchtungskammer (310) gemessen wird, einem Taupunkt, der an dem Einlass der Befeuchtungskammer (310) gemessen wird, einer Umgebungstemperatur, einem Druck der Gase innerhalb des Strömungswegs des Systems oder einer elektrischen Leistung, einem Strom oder einer Spannung, die der Heizplatte zugeführt werden, und optional wobei die Strömungsgeschwindigkeit der Gase ein Massenstrom ist, oder optional wobei die Strömungsgeschwindigkeit der Gase eine Volumenstromrate ist.

9. System nach einem von Anspruch 7 oder 8, wobei der Inspirationsschlauch (16) ein beheizter Inspirationsschlauch ist, und wobei die Steuerung (13) konfiguriert ist, um eine Heizleistung an ein Heizelement (16a) in dem Inspirationsschlauch (16) zu steuern.

10. System nach einem der Ansprüche 7 bis 9, ferner umfassend die Befeuchtungskammer (310), und/oder optional ferner umfassend eine nicht abgedichtete Patientenschnittstelle (17), und/oder optional ferner umfassend einen Strömungsgenerator (11), der sich optional in einem Gehäuse (100) der Vorrichtung befindet, und optional wobei der Strömungsgenerator (11) ein Gebläse umfasst.

11. System nach einem der Ansprüche 7 bis 10, ferner umfassend mindestens einen Temperatursensor oder Taupunktsensor an oder nahe einem Patientenende des Inspirationsschlauchs (16).

12. Verfahren zum Steuern einer Atemunterstützungsvorrichtung, wobei die Atemunterstützungsvorrichtung einen Teil eines Atemunterstützungssystems (10) bildet und konfiguriert ist, um eine Befeuchtungskammer (310) aufzunehmen, um den Strom von Gasen zu erwärmen und zu befeuchten, das Verfahren umfassend:
unter der Steuerung einer Steuereinheit (13) der Atemunterstützungsvorrichtung,
Empfangen von Daten von einem ersten Sensor und mindestens zwei anderen Sensoren, wobei der erste Sensor konfiguriert ist, um eine aktuelle Temperatur einer Heizplatte der Atemunterstützungsvorrichtung zu messen, und die mindestens zwei anderen Sensoren konfiguriert sind, um Parameter zu messen, die sich auf mindestens eines der Gase, die durch die Vorrichtung strömen, eine Umgebung oder Hardware innerhalb der Vorrichtung beziehen, wobei jeder der mindestens zwei anderen Sensoren konfiguriert ist, um einen unterschiedlichen Parameter als im Übrigen der mindestens zwei oder mehr Sensoren zu messen;
Empfangen der Benutzereingabe über eine Benutzerschnittstelle (14), wobei die Benutzereingabe mindestens eine Parametereinstellung für die Atemunterstützungsvorrichtung ändert;
Bestimmen einer Heizplattentemperaturgrenze als Reaktion auf die Benutzereingabe, die über die Benutzerschnittstelle (14) empfangen wird, und mindestens teilweise basierend auf Daten von den mindestens zwei anderen Sensoren; und
Berechnen einer maximalen Leistungsgrenze für die Heizplatte basierend mindestens teilweise auf der Heizplattentemperaturgrenze und den Daten von dem ersten Sensor, wobei die maximale Heizplattenleistungsgrenze verhindert, dass die Heizplattentemperatur die Heizplattentemperaturgrenze überschreitet.

13. Verfahren nach Anspruch 12, wobei die Leistung, die der Heizplatte bereitgestellt wird von einem Tastverhältnis abhängig ist, und/oder optional wobei die Heizplattentemperaturgrenze eine Temperatur ist, bei der die Heizplatte konfiguriert ist, um betrieben zu werden, ohne dass eine Taupunkttemperatur der Gase, die in die Atemwege des Benutzers geleitet werden, einen Wert überschreitet, der zu einer Verletzung des Patienten führen kann, und/oder optional wobei die Benutzereingabe mindestens eines der umfasst von: einer Temperatursollwertänderung, einer Taupunktsollwertänderung oder einer Strömungsgeschwindigkeitssollwertänderung.

14. Verfahren nach Anspruch 12 oder 13, ferner umfassend das Aktualisieren einer aktuellen maximalen Leistungsgrenze für die Heizplatte auf die berechnete maximale Leistungsgrenze für die Heizplatte als Reaktion darauf, dass die bestimmte Heizplattentemperaturgrenze niedriger als eine aktuelle Heizplattentemperaturgrenze ist, und/oder optional ferner umfassend das Beibehalten einer bestehenden maximalen Leistungsgrenze für die Heizplatte, es sei denn, die bestimmte Heizplattentemperaturgrenze niedriger als die aktuelle Heizplattentemperaturgrenze ist, und/oder optional wobei die aktuelle Heizplattentemperaturgrenze eine voreingestellte Temperaturgrenze ist, und optional wobei die voreingestellte Temperaturgrenze eine Temperaturgrenze ist, oberhalb derer ein Hardware-Sicherheitsmerkmal ausgelöst wird.

15. Verfahren nach einem der Ansprüche 12 bis 14, wobei die Parameter, die durch die mindestens zwei anderen Sensoren gemessen werden, mindestens zwei umfassen von: einer Strömungsgeschwindigkeit der Gase innerhalb eines Strömungswegs des Atemunterstützungssystems, einer Temperatur, die an einem Auslass der Befeuchtungskammer (310) gemessen wird, einer Temperatur, die an dem Einlass der Befeuchtungskammer (310) gemessen wird, einer Umgebungstemperatur, einem Druck der Gase innerhalb des Strömungspfads des Systems oder einer elektrischen Leistung, Stromstärke oder Spannung, die der Heizplatte zugeführt wird, und optional wobei die Strömungsgeschwindigkeit der Gase ein Massenstrom ist, oder optional wobei die Strömungsgeschwindigkeit der Gase ein Volumenstrom ist.

## Revendications

1. Dispositif d'assistance respiratoire faisant partie d'un système d'assistance respiratoire (10) qui délivre un flux de gaz à un patient, dans lequel le dispositif d'assistance respiratoire est configuré pour recevoir une chambre d'humidification (310) contenant un volume de liquide, le dispositif d'assistance respiratoire comprenant :
une plaque de dispositif de chauffage configurée pour transférer de la chaleur à la chambre d'humidification (310), dans lequel une entrée (306) de la chambre d'humidification (310) est configurée pour recevoir le flux de gaz à chauffer et à humidifier, et une sortie (308) de la chambre d'humidification (310) est configurée pour être accouplée à un tube inspiratoire (16) ;
un premier capteur configuré pour mesurer une température actuelle de la plaque de dispositif de chauffage ;
au moins un ou plusieurs autres capteurs configurés pour mesurer des paramètres relatifs à au moins l'un des gaz s'écoulant à travers le dispositif, un environnement ambiant, ou un matériel au sein du dispositif, dans lequel chacun des au moins un ou plusieurs autres capteurs est configuré pour mesurer un paramètre différent du reste des au moins un ou plusieurs autres capteurs ;
une interface utilisateur (14) configurée pour recevoir une entrée d'utilisateur ; et
un dispositif de commande (13) en communication électrique avec la plaque de dispositif de chauffage, le premier et au moins un ou plusieurs autres capteurs, et l'interface utilisateur (14), dans lequel le dispositif de commande (13) est configuré pour commander une sortie de chaleur de la plaque de dispositif de chauffage afin d'ajuster la chaleur transférée à la chambre d'humidification (310), dans lequel le dispositif de commande (13) est en outre configuré pour :
déterminer une limite de température de plaque de dispositif de chauffage (504) en réponse à l'entrée d'utilisateur reçue par l'intermédiaire de l'interface utilisateur (14) et sur la base, au moins en partie, des paramètres mesurés par l'au moins un ou plusieurs autres capteurs ; et
calculer une limite de puissance maximale (512) pour la plaque de dispositif de chauffage sur la base, au moins en partie, de la limite de température de plaque de dispositif de chauffage et de données provenant du premier capteur, dans lequel la limite de puissance de plaque de dispositif de chauffage maximale (512) empêche la température de plaque de dispositif de chauffage de dépasser la limite de température de plaque de dispositif de chauffage (504).

2. Dispositif selon la revendication 1, dans lequel la limite de température de plaque de dispositif de chauffage est une température à laquelle la plaque de dispositif de chauffage est configurée pour fonctionner de telle sorte qu'un point de rosée des gaz est inférieur à une limite pouvant entraîner une blessure pour le patient.

3. Dispositif selon la revendication 1 ou la revendication 2, dans lequel l'entrée d'utilisateur comprend au moins l'un de : un changement de point de consigne de température, un changement de point de consigne de point de rosée, un changement de point de consigne de débit, ou une quelconque combinaison de ceux-ci.

4. Dispositif selon l'une quelconque des revendications 1 à 3, dans lequel le dispositif de commande (13) est configuré pour mettre à jour une limite de puissance maximale actuelle pour la plaque de dispositif de chauffage afin qu'elle soit la limite de puissance maximale calculée pour la plaque de dispositif de chauffage en réponse au fait que la limite de température de plaque de dispositif de chauffage déterminée est inférieure à une limite de température de plaque de dispositif de chauffage actuelle, et/ou facultativement dans lequel le dispositif de commande (13) est configuré pour maintenir une limite de puissance maximale existante pour la plaque de dispositif de chauffage à moins que la limite de température de plaque de dispositif de chauffage déterminée ne soit inférieure à la limite de température de plaque de dispositif de chauffage actuelle, et/ou facultativement dans lequel la limite de température de plaque de dispositif de chauffage actuelle est une limite de température préréglée, et facultativement dans lequel la limite de température préréglée est une limite de température au-dessus de laquelle une fonction de sécurité matérielle est déclenchée.

5. Dispositif selon l'une quelconque des revendications 1 à 4, dans lequel la puissance fournie à la plaque de dispositif de chauffage dépend d'un cycle de service.

6. Dispositif selon l'une quelconque des revendications 1 à 5, dans lequel la sortie de la chambre d'humidification (310) comprend un coude de sortie amovible comportant un orifice de sortie patient (344) pour un accouplement au tube inspiratoire (16), et facultativement dans lequel l'au moins un ou plusieurs autres capteurs comprend deux autres capteurs ou plus.

7. Système d'assistance respiratoire (10) comprenant :
le dispositif d'assistance respiratoire selon l'une quelconque des revendications 1 à 6 ; et
le tube inspiratoire (16).

8. Système selon la revendication 7, dans lequel les paramètres mesurés par l'au moins un ou plusieurs autres capteurs comprennent au moins l'un de : un débit des gaz au sein d'un trajet d'écoulement du système, une température mesurée au niveau de la sortie de la chambre d'humidification, un point de rosée mesuré au niveau de la sortie (308) de la chambre d'humidification (310), une température mesurée au niveau de l'entrée de la chambre d'humidification (310), un point de rosée mesuré au niveau de l'entrée de la chambre d'humidification (310), une température ambiante, une pression des gaz au sein du trajet d'écoulement du système, ou une puissance, un courant ou une tension électrique fourni à la plaque de dispositif de chauffage, et facultativement dans lequel le débit des gaz est un débit massique, ou facultativement dans lequel le débit des gaz est un débit volumétrique.

9. Système selon l'une quelconque de la revendication 7 ou de la revendication 8, dans lequel le tube inspiratoire (16) est un tube inspiratoire chauffé, et dans lequel le dispositif de commande (13) est configuré pour commander une sortie de chaleur vers un élément chauffant (16a) dans le tube inspiratoire (16).

10. Système selon l'une quelconque des revendications 7 à 9, comprenant en outre la chambre d'humidification (310), et/ou comprenant en outre facultativement une interface patient non étanche (17), et/ou comprenant en outre facultativement un générateur de flux (11) facultativement situé dans un boîtier (100) du dispositif, et facultativement dans lequel le générateur de flux (11) comprend une soufflante.

11. Système selon l'une quelconque des revendications 7 à 10, comprenant en outre au moins l'un d'un capteur de température ou d'un capteur de point de rosée au niveau ou à proximité d'une extrémité côté patient du tube inspiratoire (16).

12. Procédé de commande d'un dispositif d'assistance respiratoire, dans lequel le dispositif d'assistance respiratoire fait partie d'un système d'assistance respiratoire (10) et est configuré pour recevoir une chambre d'humidification (310) pour chauffer et humidifier le flux de gaz, le procédé comprenant :
sous la commande d'un dispositif de commande (13) du dispositif d'assistance respiratoire,
la réception de données provenant d'un premier capteur et d'au moins deux autres capteurs, le premier capteur étant configuré pour mesurer une température actuelle d'une plaque de dispositif de chauffage du dispositif d'assistance respiratoire et les au moins deux autres capteurs étant configurés pour mesurer des paramètres relatifs à au moins l'un des gaz s'écoulant à travers le dispositif, un environnement ambiant ou un matériel au sein du dispositif, dans lequel chacun des au moins deux autres capteurs est configuré pour mesurer un paramètre différent de celui du reste des au moins deux capteurs ou plus ;
la réception d'une entrée d'utilisateur, par l'intermédiaire d'une interface utilisateur (14), dans lequel l'entrée d'utilisateur modifie au moins un réglage de paramètre pour le dispositif d'assistance respiratoire ;
la détermination d'une limite de température de plaque de dispositif de chauffage en réponse à l'entrée d'utilisateur reçue par l'intermédiaire de l'interface utilisateur (14) et sur la base, au moins en partie, de données provenant des au moins deux autres capteurs ; et
le calcul d'une limite de puissance maximale pour la plaque de dispositif de chauffage sur la base, au moins en partie, de la limite de température de plaque de dispositif de chauffage et des données provenant du premier capteur, dans lequel la limite de puissance maximale de plaque de dispositif de chauffage empêche la température de plaque de dispositif de chauffage de dépasser la limite de température de plaque de dispositif de chauffage.

13. Procédé selon la revendication 12, dans lequel la puissance fournie à la plaque de dispositif de chauffage dépend d'un cycle de service, et/ou facultativement dans lequel la limite de température de plaque de dispositif de chauffage est une température à laquelle la plaque de dispositif de chauffage est configurée pour fonctionner sans qu'une température de point de rosée des gaz dirigés dans les voies respiratoires de l'utilisateur ne dépasse une valeur pouvant entraîner une blessure pour le patient, et/ou facultativement dans lequel l'entrée d'utilisateur comprend au moins l'un de : un changement de point de consigne de température, un changement de point de consigne de point de rosée, ou un changement de point de consigne de débit.

14. Procédé selon la revendication 12 ou la revendication 13, comprenant en outre la mise à jour d'une limite de puissance maximale actuelle pour la plaque de dispositif de chauffage pour qu'elle soit la limite de puissance maximale calculée pour la plaque de dispositif de chauffage en réponse au fait que la limite de température de plaque de dispositif de chauffage déterminée est inférieure à une limite de température de plaque de dispositif de chauffage actuelle, et/ou comprenant en outre facultativement le maintien d'une limite de puissance maximale existante pour la plaque de dispositif de chauffage à moins que la limite de température de plaque de dispositif de chauffage déterminée ne soit inférieure à la limite de température de plaque de dispositif de chauffage actuelle, et/ou facultativement dans lequel la limite de température de plaque de dispositif de chauffage actuelle est une limite de température préréglée, et facultativement dans lequel la limite de température préréglée est une limite de température au-dessus de laquelle une fonction de sécurité matérielle est déclenchée.

15. Procédé selon l'une quelconque des revendications 12 à 14, dans lequel les paramètres mesurés par les au moins deux autres capteurs comprennent au moins deux éléments de : un débit des gaz au sein d'un trajet d'écoulement du système d'assistance respiratoire, une température mesurée au niveau d'une sortie de la chambre d'humidification (310), une température mesurée au niveau de l'entrée de la chambre d'humidification (310), une température ambiante, une pression des gaz au sein du trajet d'écoulement du système, ou une puissance, un courant ou une tension électrique fourni à la plaque de dispositif de chauffage, et facultativement dans lequel le débit des gaz est un débit massique, ou facultativement dans lequel le débit des gaz est un débit volumétrique.
